# EUROPEAN PATENT SPECIFICATION

(11) **EP 3 536 790 B1**
(45) Date of publication and mention of the grant of the patent: **18.08.2021**
(21) Application number: 17866684.8
(22) Date of filing: 03.11.2017
(51) Int. Cl.: C12N 15/79, C12P 21/00, C07K 14/485, A61K 8/64, A61Q 19/00, A61Q 19/08

(54) **VECTOR FOR EXPRESSING HUMAN EPIDERMAL GROWTH FACTOR OR SKIN-PERMEABLE HUMAN EPIDERMAL GROWTH FACTOR, AND MICROALGAE TRANSFORMED WITH VECTOR**
VEKTOR ZUR EXPRESSION DES MENSCHLICHEN EPIDERMALEN WACHSTUMSFAKTORS ODER HAUTPERMEABLEN EPIDERMALEN WACHSTUMSFAKTORS UND MIT DEM VEKTOR UMGEWANDELTE MIKROALGEN
VECTEUR D'EXPRESSION DU FACTEUR DE CROISSANCE ÉPIDERMIQUE HUMAIN OU DU FACTEUR DE CROISSANCE ÉPIDERMIQUE HUMAIN PERMÉABLE AU NIVEAU DE LA PEAU, ET MICROALGUE TRANSFORMÉE AVEC LE VECTEUR

(30) Priority: 04.11.2016 KR 20160146839; 02.11.2017 KR 20170145448
(43) Date of publication of application: 11.09.2019
(73) Proprietor: Korea Institute of Science and Technology, Seoul 02792 (KR)
(72) Inventor: PAN, Cheol-Ho, Gangneung-si Gangwon-do 25451 (KR); SHIN, Bok-Kyu, Gangneung-si Gangwon-do 25451 (KR); ERDENE-OCHIR, Erdenedolgor, Gangneung-si Gangwon-do 25451 (KR); KIM, Sang Min, Seoul 02792 (KR)
(74) Representative: advotec.
(86) International application number: PCT/KR2017/012437
(87) International publication number: WO 2018/084650

(56) References cited:
- EP-A1- 0 652 954
- WO-A1-90/10697
- WO-A1-2014/030165
- KR-A- 940 024 067
- KR-A- 20040 031 815
- KR-A- 20040 056 365
- KR-B1- 100 961 528
- KR-B1- 101 574 204
- KR-B1- 101 618 767
- US-B1- 6 589 540
- Anonymous: "EM_EST:CT946228", , 8 April 2007 (2007-04-08), XP055692167, Retrieved from the Internet: URL:http://ibis.internal.epo.org/exam/dbfe tch.jsp?id=EM_EST:CT946228 [retrieved on 2020-05-06] -& BOWLER CHRIS ET AL: "The Phaeodactylum genome reveals the evolutionary history of diatom genomes", NATURE, MACMILLAN JOURNALS LTD, LONDON, vol. 456, no. 7219, 13 November 2008 (2008-11-13), pages 239-244, XP002616144, ISSN: 0028-0836
- AMIR ATA SAEI ET AL: "Haematococcus as a promising cell factory to produce recombinant pharmaceutical proteins", MOLECULAR BIOLOGY REPORTS ; AN INTERNATIONAL JOURNAL ON MOLECULAR AND CELLULAR BIOLOGY, KLUWER ACADEMIC PUBLISHERS, DO, vol. 39, no. 11, 26 June 2012 (2012-06-26) , pages 9931-9939, XP035117695, ISSN: 1573-4978, DOI: 10.1007/S11033-012-1861-Z
- AKBARI FARIBA ET AL: "The potential of transgenic green microalgae; a robust photobioreactor to produce recombinant therapeutic proteins", WORLD JOURNAL OF MICROBIOLOGY AND BIOTECHNOLOGY, RAPID COMMUNICATIONS OF OXFORD, OXFORD, GB, vol. 30, no. 11, 13 August 2014 (2014-08-13), pages 2783-2796, XP035400449, ISSN: 0959-3993, DOI: 10.1007/S11274-014-1714-0 [retrieved on 2014-08-13]
- WEIQI FU ET AL: "Algal Cell Factories: Approaches, Applications, and Potentials", MARINE DRUGS, vol. 14, no. 12, 13 December 2016 (2016-12-13), page 225, XP055692120, DOI: 10.3390/md14120225
- MIFLIN, B. J. et al.: "The Role of Glutamine Synthetase and Glutamate Dehydrogenase in Nitrogen Assimilation and Possibilities for Improvement in the Nitrogen Utilization of Crops", Journal of Experimental Botany, vol. 53, no. 370, 1 April 2002 (2002-04-01), pages 979-987, XP002435264, ISSN: 0022-0957, DOI: 10.1093/jexbot/53.370.979

## Description

### [Technical Field]

The present specification relates to a vector for expressing a human epidermal growth factor or a skin-permeable human epidermal growth factor and microalgae transformed with the vector, particularly to a vector comprising the base sequence represented by SEQ ID NO: 2 or 5 and *Phaeodactylum tricornutum* transformed with the vector.

### [Background Art]

Human epidermal growth factor (hEGF) is a functional protein used as a main ingredient of wrinkle-improving cosmetics. Currently, hEGF is mostly produced from *E. coli* for use. However, *E. coli* contains endotoxin and thus poses a risk.

Therefore, there is a need for a material that can replace existing hEGF raw materials produced from *E. coli,* and in which the risk of endotoxin is fundamentally eliminated.

US patent 6 589 540 B1 provides a cosmetic composition for skin care, which enhances the effect of retinol, one of the conventional skin care ingredients, and also alleviates the adverse effects of retinol. Thus, a skin care cosmetic composition is provided which enhances the effects of retinol, such as the treatment of acne, the improvement of skin wrinkles, age spots, freckles, blotches or other pigmentation, and the moisturizing of skin, and also alleviates adverse effects of retinol, such as skin irritation. Thus, it has been found that epidermal growth factor (EGF) remarkably enhances the effect of retinol used in topical therapeutics and cosmetics, and also effectively alleviates the skin irritations caused by retinol.

Patent application KR 2004 003 18 15 A relates to a skin cosmetic composition for protection, including man epidermal growth factor (human epidermal growth factor, hEGF) and Lactobacillus cain (Lactokine) for cosmetic compositions for skin protection. Thus, the cosmetic composition comprising the Lactobacillus cain as a component that maximizes the capabilities of hEGF, and to minimize the side effects, has excellent skin wrinkle improvement properties.

Patent application KR 2004 005 63 65 A refers to a cosmetic composition comprising recombinant human epidermal growth factor (rhEGF). The epidermal growth factor has in the total cosmetic composition a content of 0.00000001 to 0.001 % by weight of itself or nano-bit processed epidermal growth factor.

Patent application EP 0 652 954 A1 relates to a novel gene coding human epidermal growth factor (hEGF) and a process for preparing the same employing a recombinant expression vector therefor.

International patent application WO 90/10697 A1 also refers to epidermal growth factor (EGF), a naturally occurring, relatively short single-chain polypeptide which is prepared by growing methylotrophic yeast transformants containing in the genome at least one copy of a DNA sequence operably encoding EGF, in operational association with a DNA sequence encoding the yeast α-mating factor pre-pro sequence, both under the regulation of a promotor region of a gene of a methylotrophic yeast, under conditions allowing expression of said DNA sequences, and secretion of EGF into the culture medium. Further disclosed are novel DNA fragments and novel recombinant yeast strains.

Patent KR 100 961 528 B1 refers to a mass-producing method of active human epidermal growth factor from E. coli which is provided to over-express human epidermal growth factor protein in active form by fusing with fusion peptide or protein. Thus, a production method of active human epidermal growth factor comprises a step of culturing E. coli host cells transformed with a recombinant vector to express fusion protein which peptide or protein selected from a group of signal sequences which consists of human epidermal growth factor and synuclein fragment of N-terminal or C-terminal of human epidermal growth factor, maltose binding protein, disulfide bond A, disulfide bond C, pectate lyase B; and a step of separating the fusion protein from culture media.

### [Summary of Invention]

### [Technical Problem]

In view of the above problems, the present inventors have conducted researches on a method capable of effectively producing a hEGF protein while fundamentally blocking the risk of endotoxicity, which led to the present invention.

In one aspect, an object of the present invention is to provide a vector comprising a base sequence coding for a human epidermal growth factor (hEGF) of SEQ ID NO: 2.

In one aspect, an object of the present invention is to provide a vector comprising a base sequence coding for a skin-permeable human epidermal growth factor (PTD-hEGF) of SEQ ID NO: 5.

In one aspect, an object of the present invention is to provide a vector comprising a base sequence represented by SEQ ID NO: 6 or SEQ ID NO: 7.

In another aspect, an object of the present invention is to provide microalgae transformed with the vector.

In another aspect, an object of the present invention is to provide a method for producing a hEGF protein or a PTD-hEGF by using the transformed microalgae.

### [Solution to Problem]

In one aspect, the present invention provides a vector comprising a promoter of SEQ ID NO: 1; and a human epidermal growth factor (hEGF) exon, which includes a gene coding for hEGF.

In one aspect of the present invention, the hEGF exon may further comprise a His-tag sequence and a TEV site sequence.

In one aspect of the invention, the hEGF exon may further comprise a PTD sequence that is a skin-permeable peptide sequence.

In one aspect of the present invention, the hEGF exon may be a base sequence represented by SEQ ID NO: 2.

In one aspect of the present invention, the hEGF exon may be a base sequence represented by SEQ ID NO: 5.

In one aspect of the invention, the vector may comprise a sequence represented by SEQ ID NO: 6.

In one aspect of the invention, the vector may comprise a sequence represented by SEQ ID NO: 7.

In another aspect, the present invention provides microalgae transformed with a vector according to any of the above.

In another aspect of the invention, the microalgae may be *Phaeodactylum tricornutum.*

In another aspect of the invention, the microalgae may be deposited with the accession numbers KCTC 13382BP or KCTC 13383BP.

In another aspect, the present invention provides a method for producing a human epidermal growth factor (hEGF) protein by using the transformed microalgae.

In another aspect of the present invention, the human epidermal growth factor (hEGF) protein may be a skin-permeable human epidermal growth factor (PTD-hEGF) protein.

A cosmetic composition containing the hEGF or PTD-hEGF protein produced according to the production method is described.

The cosmetic composition may be a cosmetic composition for improving wrinkles.

A method for improving wrinkles comprising the step of administering the hEGF or PTD-hEGF protein produced according to the production method to a subject in need thereof is described.

Disclosed is the use of the hEGF or PTD-hEGF protein produced according to the production method for use in wrinkle improvement.

Furthermore disclosed is the use of the hEGF or PTD-hEGF protein produced according to the production method for use in the preparation of a composition for improving wrinkles.

In another aspect, the present invention provides a kit comprising the transformed microalgae.

### [Advantageous Effects of Invention]

The microalgae strain according to one aspect of the present invention is capable of producing a hEGF or PTD-hEGF protein without endotoxicity.

The hEGF or PTD-hEGF protein produced according to one aspect of the present invention can replace existing hEGF proteins produced by *E. coli.*

The hEGF or PTD-hEGF protein produced according to one aspect of the present invention is excellent in skin permeability.

The cosmetic composition not covered by the claims containing the hEGF or PTD-hEGF protein produced according to one aspect of the present invention has an excellent effect of improving wrinkles.

### [Brief Description of Drawings]

FIG. 1 is a diagram showing a vector construct according to an example of the present invention, where GS denotes glutamine synthetase (promoter), TEV denotes tobacco etch virus (TEV cleavage site), 3-UTR denotes 3 terminal untranslated region (terminator), FcpB denotes fucoxanthin/chlorophyll binding protein B (promoter), and shble denotes zeocin resistance gene (selection marker).
FIG. 2 shows the results of confirmation of gene insertion by cDNA-PCR. FIG. 2a shows the cDNA-PCR results of hEGF and FIG. 2b shows the cDNA-PCR results of PTD-hEGF.
FIG. 3 shows the results of confirmation of mRNA expression. FIG. 3a shows the qRT-PCR results of hEGF and FIG. 3b shows the qRT-PCR results of PTD-hEGF.
FIG. 4 shows the results of confirmation of protein expression, including the results of SDS-PAGE (top) and Western blot (bottom).
FIG. 5 shows the results of MTT analysis of Experimental Example 4 of the present invention for measuring cell activity.

### [Description of Embodiments]

### [Embodiments]

"Human epidermal growth factor (hEGF or EGF)", which is known as urogastrone, is a polypeptide with a molecular weight of 6045 Da having 53 amino acids and three disulfide bonds. It is known to stimulate the regeneration and differentiation of the enteric mucous membranes, corneal epidermal tissues and lung epidermal tissues, thereby growing the epidermis, promoting angiogenesis, increasing wound healing power, and inhibiting gastric acid secretion. Due to these functions, hEGF has been developed and widely used as a wound medicine. Recently, hEGF is drawing attention as a raw material of functional cosmetics because it has been proven to have wrinkle improvement and anti-aging effects.

According to one aspect of the present invention, hEGF produced from microalgae can fundamentally eliminate the risk of endotoxin and can be used as a substitute for existing hEGF raw materials produced from *E. coli.* In addition, a skin-permeable human epidermal growth factor (PTD-hEGF) which has increased skin permeability and thus enhanced efficiency can also be produced from microalgae. These hEGF and PTD-hEGF produced from microalgae can be used as raw materials of cosmetics in line with recent trends in cosmetics, which are eco-friendliness and naturalness.

Hereinafter, the present invention will be described in detail.

In one aspect, the present invention provides a vector comprising a promoter of SEQ ID NO: 1; and a human epidermal growth factor (hEGF) exon, which includes a gene coding for hEGF.

In one aspect of the present invention, the hEGF exon may further comprise a His-tag sequence and a TEV site sequence.

In one aspect of the invention, the hEGF exon may further comprise a PTD sequence that is a skin-permeable peptide sequence.

In one aspect of the present invention, the hEGF exon may be a base sequence represented by SEQ ID NO: 2.

In one aspect of the present invention, the hEGF exon may be a base sequence represented by SEQ ID NO: 5.

In one aspect of the invention, the vector may comprise a sequence represented by SEQ ID NO: 6. Specifically, SEQ ID NO: 6 comprises a GS promoter sequence, a His-tag sequence, a TEV sequence, an EGF sequence, a 3'-UTR sequence, and shble cassette.

In one aspect of the present invention, the vector may comprise a base sequence represented by SEQ ID NO: 7. Specifically, the base sequence represented by SEQ ID NO: 7 comprises a GS promoter sequence, a His-tag sequence, a TEV sequence, a PTD sequence, an EGF sequence, a 3'-UTR sequence, and shble cassette.

The shble cassette may comprise FcpB, shble, and 3'-UTR.

In another aspect, the present invention provides microalgae transformed with a vector according to any of the above.

In another aspect of the invention, the microalgae may be *Phaeodactylum tricornutum.* The *Phaeodactylum tricornutum* is readily available from foreign microalgae banks. Specifically, *Phaeodactylum tricornutum* (Bohlin) can be purchased from UTEX, a depository authority (strain number: UTEX 646). Also, *Phaeodactylum tricornutum* (Bohlin) can be purchased from CCAP, a depository authority (strain number: CCAP1052/6) and from CCMP, a depository authority (strain number: CCMP2559).

In another aspect of the invention, the microalgae may be deposited with the accession numbers KCTC 13382BP or KCTC 13383BP, although not limited thereto. Here, the microalgae deposited with the accession number KCTC 13382BP are microalgae capable of producing the target protein EGF, and the microalgae deposited with the accession number KCTC 13383BP are microalgae capable of producing the target protein PTD-EGF.

In another aspect, the present invention provides a method for producing a human epidermal growth factor (hEGF) protein by using the transformed microalgae.

In another aspect of the present invention, the human epidermal growth factor (hEGF) protein may be a skin-permeable human epidermal growth factor (PTD-hEGF) protein.

A cosmetic composition containing the hEGF or PTD-hEGF protein produced according to the production method is disclosed.

The cosmetic composition may be a cosmetic composition for improving wrinkles.

In another aspect, the present invention provides a kit comprising the transformed microalgae.

### [Examples]

Hereinafter, the present invention will be described in detail by way of examples. It will be apparent to those skilled in the art that these examples are for illustrative purposes only.

### Example 1: Promoter search and selection

### (1) Experimental method

A protein having a high expression level in the microalgae *Phaeodactylum tricornutum* was selected and the promoter portion thereof was used. Specifically, the promoter was searched for by the following method:
- *P. tricornutum* (Bohlin) UTEX 646 was purchased from UTEX (utex.org).
- The *P. tricornutum* strain was cultured in 200 mL of F/2 medium (50% artificial seawater).
- 50 mL of the culture fluid was taken and centrifuged to obtain a cell pellet, which was then subjected to cell lysis by ultrasonication.
- After removing the lysis debris by centrifugation (17000 rpm, 15 min), the supernatant was taken, and protein separation was performed by SDS-PAGE electrophoresis.
- After electrophoresis, proteins were extracted again from five with strong protein bands and subjected to LC-MS/MS analysis.
- A protein having a high expression level in *P*. *tricornutum* was selected, and the promoter region was selected from the genomic DNA information.

### (2) Experimental results - Selection of protein expression promoter: Glutamine synthetase promoter

- From the analysis results of proteins overexpressed in *P. tricornutum,* glutamine synthetase was found to be most overexpressed. Thus, glutamine synthetase promoter was selected for use.

### Example 2: Construction of vectors

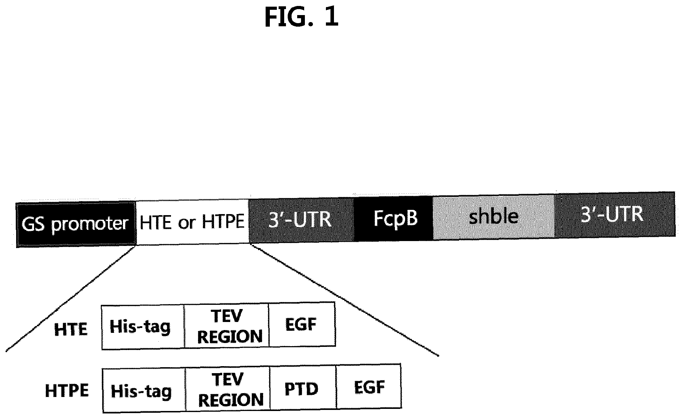

The vector was constructed by the following method:
- 50 mL of *P. tricornutum* culture fluid cultured in F/2 medium (50% artificial seawater) was taken to obtain a cell pellet, from which genomic DNA (gDNA) was extracted.
- DNA of the promoter portion selected in Example 1 was obtained from the extracted gDNA through PCR.
- EGF expression vectors containing the promoter portion were constructed as shown in FIG. 1 to construct a vector capable of producing the target protein EGF and a vector capable of producing PTD-EGF.
- Sequences constituting the vectors were as shown in Table 1 below.

**Table 1.**

| | |
|---|---|
| SEQ ID NO: 1 | GS promoter |
| SEQ ID NO: 2 | His-TEV-EGF |
| SEQ ID NO: 3 | 3'-UTR |
| SEQ ID NO: 4 | Shble cassette (comprising FcpB, shble, and 3'-UTR) |
| SEQ ID NO: 5 | His-TEV-PTD-EGF |

### Example 3: Microalgae transformation

Microalgae transformation was performed by the following method:
- The vectors for producing the target proteins in Example 2 were obtained in large quantities using *E. coli* DH5a (NEB® 5-alpha Competent *E. coli).*
- High purity vectors for transformation of *P*. *tricornutum* were prepared using the miniprep method and phenol/chloroform DNA purification method.
- The high purity vectors were coated on gold nanoparticles and *P. tricornutum* was transformed by transferring the target protein expression vectors to *P*. *tricornutum* through particle bombardment.
- The transformants were selected in F/2 medium containing the antibiotic zeocin (100 µg/mL).
- The transformed microalgae finally selected from the selected microalgae through the Experimental Examples described below were deposited with the accession numbers KCTC 13382BP and KCTC 13383BP, respectively. The microalgae deposited with the accession number KCTC 13382BP is microalgae *(Phaeodactylum tricornutum,* PT-GSp-O-HTE) capable of producing the target protein EGF, and the microalgae deposited with the accession number KCTC 13383BP is microalgae capable of producing the target protein PTD-EGF *(Phaeodactylum tricornutum,* PT-GSp-O-HTPE).

### Experimental Example: Confirmation of expression of hEGF and PTD-hEGF

Expression of hEGF and PTD-hEGF from the *P. tricornutum* transformants selected in Example 3 was confirmed from mRNA expression and protein expression of the inserted gene, specifically in Experimental Examples 1 to 3 below.

### Experimental Example 1: Confirmation of gene insertion by cDNA PCR

### (1) Experimental method

In Example 3, 25 transformants for each of hEGF and PTD-hEGF were selected from the microalgae transformants obtained through the transformation, and then cultured. During the culture, ten transformants growing well were selected from each and cDNA PCR was performed.
- For cDNA PCR, RNA was extracted from microalgae transformants using RNeasy Plant Mini Kit (QIAGEN), and then cDNA was synthesized using QuantiTect Reverse Transcription Kit (QIAGEN).
- The obtained cDNA, the primer corresponding to the EGF base sequence (identifying EGF mRNA), and the primer corresponding to the PTD-EGF base sequence were mixed, and PCR was performed using Taq polymerase (i-StarTaq™ DNA Polymerase).

A blank transformant transformed with a vector lacking the EGF base sequence was used as the control group.

### (2) cDNA-PCR results

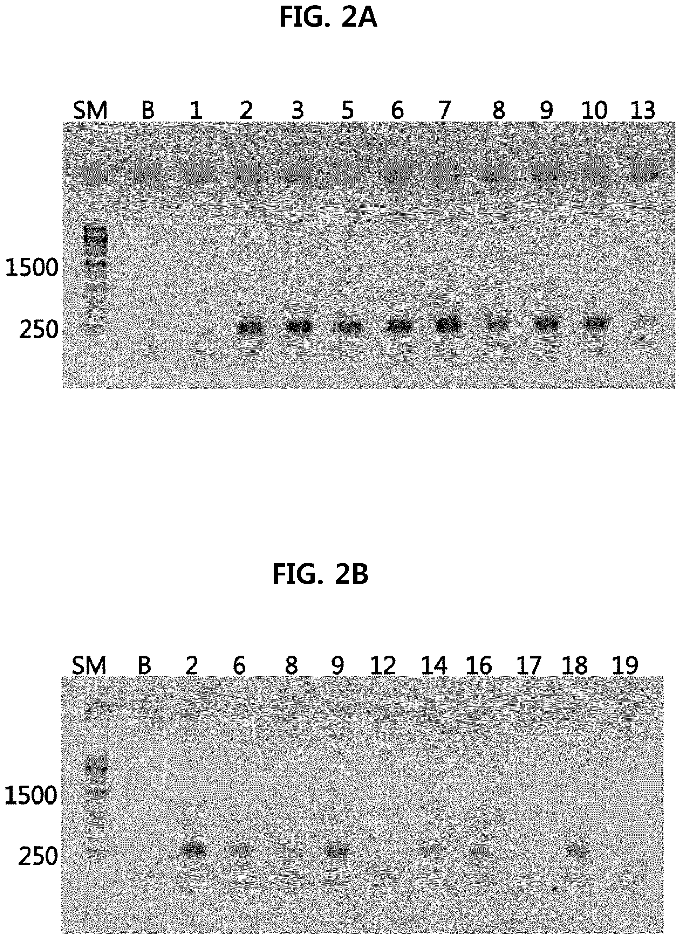

- The final length of the inserted gene was 243 bp for HIS-TEV-hEGF and 297 bp for HIS-TEV-PTD-hEGF.
- The DNA electrophoresis results are shown in FIG. 2. FIG. 2a shows the cDNA-PCR results of hEGF and FIG. 2b shows the cDNA-PCR results of PTD-hEGF. The results showed that the target gene bands were found at similar positions.
- A transformant (Blank) inserted with an empty vector in which the target genes were not inserted was used as the control group. The target gene bands were not found in the control group.

### Experimental Example 2: Confirmation of the expression level of messenger RNA (mRNA) by quantitative real-time PCR (qRT-PCR)

### (1) Experimental method

- Expression of hEGF and PTD-hEGF as in cDNA PCR of Experimental Example 1; qRT-PCR was performed using 10 cDNAs of each of the microalgae transformants.
- The obtained cDNA, the primer corresponding to the EGF base sequence (identifying EGF mRNA) and the primer corresponding to the PTD-EGF base sequence were mixed, and qRT-PCR was performed using LightCycler® 480 SYBR Green I Master Kit (Roche).
- LightCycler® 480 Instrument II was used for qRT-PCR.
- Wild type and a blank transformant transformed with a vector lacking the EGF base sequence was used as the control groups.

### (2) qRT-PCR results

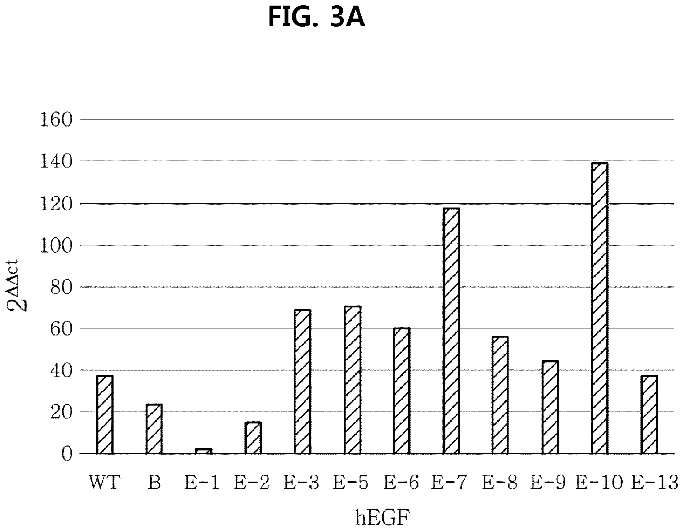

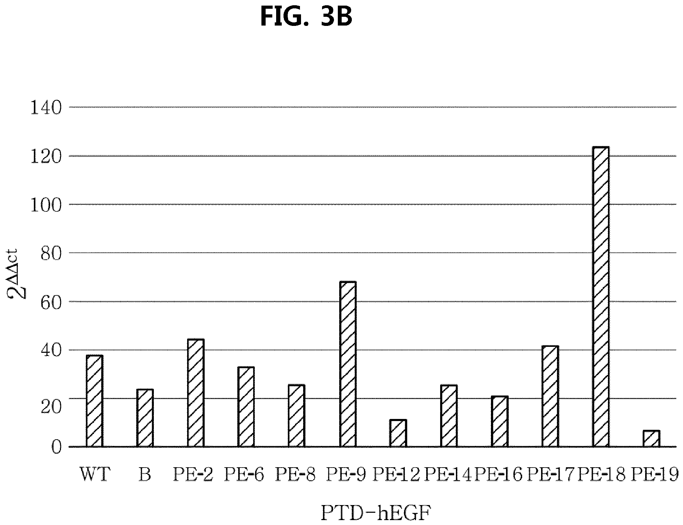

The results are shown in FIG. 3. FIG. 3a shows the qRT-PCR results of hEGF and FIG. 3b shows the qRT-PCR results of PTD-hEGF.
- It was found that hEGF mRNA expression level was higher in the transformants producing hEGF and PTD-hEGF than in the WT (wild type) and the Blank, which are used as the control groups.

### Experimental Example 3: Confirmation of expression of the target proteins by Western blot using an EGF-antibody

### (1) Experimental method

- In Experimental Example 2, *P. tricornutum* transformants having a high mRNA expression level were selected and subjected to Western blot using an EGF antibody (ab9695, Abcam) .
- In Example 3, 50 mL of the transformant culture fluids was taken to obtain cell pellets, which were then subjected to cell lysis by ultrasonication.
- The supernatant was subjected to SDS-PAGE and Western blot to confirm the expression of EGF.
- A blank transformant transformed with a vector lacking the EGF base sequence was used as the control group.

### (2) Western blot results

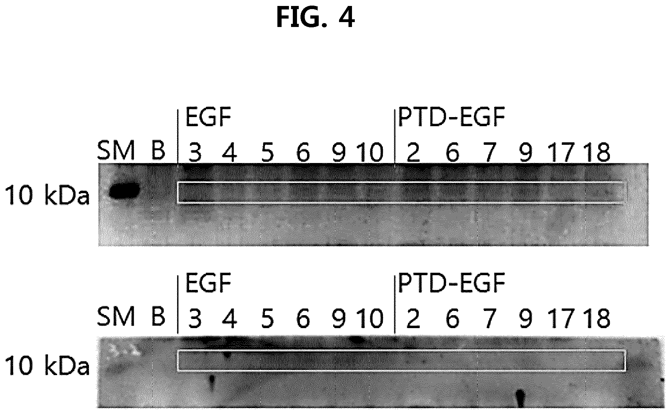

- The results are shown in FIG. 4. FIG. 4 shows the results of measurement of protein expression, including the results of SDS-PAGE (top) and Western blot (bottom).
- The target protein bands were found at the protein molecular weight of around 10 kDa: 9.25 kDa for His-TEV-hEGF and 11.64 kDa for His-TEV-PTD-hEGF.
- Such protein expression was not found in the Blank as the control group.

### Experimental Example 4: MTT assay (cell activity measurement)

### (1) Experimental method

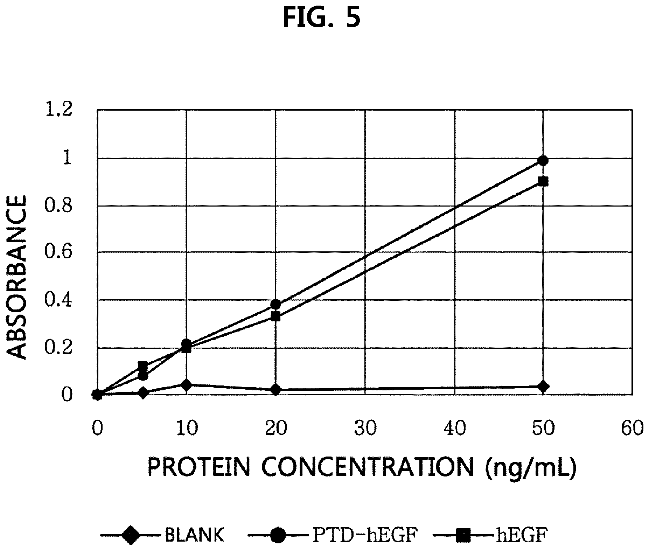

- In Experimental Examples 2 and 3, 50 mL of the transformant culture fluids having high hEGF and PTD-hEGF expression levels were taken to obtain cell pellets, which were then subjected to cell lysis by ultrasonication.
- The debris was removed by centrifugation (17000 rpm, 15 min), and the supernatants were taken.
- The total protein concentration of the supernatants was measured by Bradford assay.
- hEGF expression was measured by MTT assay (cell activity test). MTT analysis was performed by the following experimental method.
- BALB/3T3 cells (ATCC; purchased from the Global Bioresource Center) were seeded into a 96-well plate, placed in a CO₂ incubator for 24 hours, and then washed with PBS on the following day.
- The cells were treated with varying concentrations of the protein extracts, left in a CO₂ incubator for 24 hours, and then treated with MTT reagent (EZ-Cytox, DOGEN) on the following day.
- After reaction in a CO₂ incubator for 3 hours, the supernatants were removed, 100ul of DMSO was added, and then the absorbance (540 nm) was measured.
- The Experimental results are shown in FIG. 5. hEGF promoted the differentiation and activity of human epithelial cells. From the measurement results of cell activity through MTT assay, it was found that treatment with hEGF and PTD-hEGF increased the activity of human epithelial cells.
- When the cell extracts of the *P. tricornutum* transformant producing hEGF and of the *P. tricornutum* transformant producing PTD-hEGF were added, the cell activity was higher than that of the control group.
- In addition, treatment with the cell extract of the transformant producing PTD-hEGF was found to be more effective in enhancing cell activity than treatment with the cell extract of the transformant producing hEGF, indicating that the efficiency was increased by enhancement of permeation by PTD.

## Claims

1. A vector, comprising:
a promoter of SEQ ID NO: 1; and
a human epidermal growth factor (hEGF) exon, which includes a gene coding for hEGF.

2. The vector according to claim 1,
wherein the hEGF exon further comprises a His-tag sequence and a TEV site sequence.

3. The vector according to any one of claims 1 and 2,
wherein the hEGF exon further comprise a PTD sequence that is a skin-permeable peptide sequence.

4. The vector according to any one of claims 1 and 2,
wherein the hEGF exon is a base sequence represented by SEQ ID NO: 2.

5. The vector according to any one of claims 1, 2 and 4,
wherein the vector comprises a base sequence represented by SEQ ID NO: 6.

6. The vector according to any one of claims 1 to 3,
wherein the hEGF exon is a base sequence represented by SEQ ID NO: 5.

7. The vector according to any one of claims 1, 2, 3 and 6,
wherein the vector comprises a sequence represented by SEQ ID NO: 7.

8. Microalgae transformed with a vector according to any one of claims 1 to 7.

9. The microalgae according to claim 8,
wherein the microalgae are *Phaeodactylum tricornutum.*

10. The microalgae according to any one of claims 8 and 9,
wherein the microalgae are deposited with the accession numbers KCTC 13382BP or KCTC 13383BP.

11. A method for producing a human epidermal growth factor (hEGF) protein by using the transformed microalgae according to any one of claims 8 to 10.

12. The method according to claim 11,
wherein the human epidermal growth factor (hEGF) protein is a skin-permeable human epidermal growth factor (PTD-hEGF) protein.

13. A kit comprising the transformed microalgae according to any one of claims 8 to 10.

## Patentansprüche

1. Vektor, umfassend:
einen Promotor der SEQ ID Nr.: 1; und
ein hEGF-Exon, das ein für hEGF (humaner epidermaler Wachstumsfaktor) codierendes Gen enthält.

2. Vektor nach Anspruch 1,
wobei das hEGF-Exon des Weiteren eine His-Tag-Sequenz und eine TEV-Stellen-Sequenz umfasst.

3. Vektor nach Anspruch 1 oder 2,
wobei das hEGF-Exon des Weiteren eine PTD-Sequenz, die eine hautgängige Peptidsequenz ist, umfasst.

4. Vektor nach Anspruch 1 oder 2,
wobei das hEGF-Exon eine durch SEQ ID Nr.: 2 dargestellte Basensequenz ist.

5. Vektor nach einem der Ansprüche 1, 2 und 4,
wobei der Vektor eine durch SEQ ID Nr.: 6 dargestellte Basensequenz umfasst.

6. Vektor nach einem der Ansprüche 1 bis 3,
wobei das hEGF-Exon eine durch SEQ ID Nr.: 5 dargestellte Basensequenz ist.

7. Vektor nach einem der Ansprüche 1, 2, 3 und 6,
wobei der Vektor eine durch SEQ ID Nr.: 7 dargestellte Basensequenz umfasst.

8. Mikroalgen, die mit einem Vektor transformiert sind, nach einem der Ansprüche 1 bis 7.

9. Mikroalgen nach Anspruch 8,
wobei es sich bei den Mikroalgen um *Phaeodactylum tricornutum* handelt.

10. Mikroalgen nach Anspruch 8 oder 9,
wobei die Mikroalgen mit der Zugangsnummer KCTC 13382BP oder KCTC 13383BP hinterlegt sind.

11. Verfahren zur Herstellung eines hEGF-Proteins unter Verwendung der transformierten Mikroalgen nach einem der Ansprüche 8 bis 10.

12. Verfahren nach Anspruch 11,
wobei das hEGF-Protein ein hautgängiges hEGF-Protein (PTD-hEGF-Protein) ist.

13. Kit, die transformierten Mikroalgen nach einem der Ansprüche 8 bis 10 umfassend.

## Revendications

1. Vecteur, comprenant :
un promoteur de la SEQ ID n° 1 ; et
un exon du facteur de croissance épidermique humain (hEGF) qui comprend un gène codant pour le hEGF.

2. Vecteur selon la revendication 1,
dans lequel l'exon du hEGF comprend en outre une séquence de His-tag et une séquence du site du TEV.

3. Vecteur selon la revendication 1 ou 2,
dans lequel l'exon du hEGF comprend en outre une séquence de PTD qui est une séquence peptidique apte à pénétrer la peau.

4. Vecteur selon la revendication 1 ou 2,
dans lequel l'exon du hEGF est une séquence de bases représentée par la SEQ ID n° : 2.

5. Vecteur selon l'une quelconque des revendications 1, 2 et 4,
dans lequel le vecteur comprend une séquence de bases représentée par la SEQ ID n° : 6.

6. Vecteur selon l'une quelconque des revendications 1 à 3,
dans lequel l'exon du hEGF est une séquence de bases représentée par la SEQ ID n° : 5.

7. Vecteur selon l'une quelconque des revendications 1, 2, 3 et 6,
dans lequel le vecteur comprend une séquence de bases représentée par la SEQ ID n° : 7.

8. Microalgues transformées avec un vecteur selon l'une quelconque des revendications 1 à 7.

9. Microalgues selon la revendications 8,
dans lesquelles les microalgues sont *Phaeodactylum tricornutum.*

10. Microalgues selon la revendications 8 ou 9,
dans lesquelles les microalgues sont déposées avec le numéro d'accès KCTC 13382BP ou KCTC 13383BP.

11. Procédé pour produire une protéine du facteur de croissance épidermique humain (hEGF) en utilisant les microalgues transformées selon l'une quelconque des revendications 8 à 10.

12. Procédé selon la revendication 11,
dans lequel la protéine du facteur de croissance épidermique humain (hEGF) est une protéine du facteur de croissance épidermique humain apte à pénétrer la peau (PTD-hEGF).

13. Trousse, comprenant les microalgues transformées selon l'une quelconque des revendications 8 à 10.


<110> KOREA INSTITUTE OF SCIENCE AND TECHNOLOGY
<120> A vector for expressing hEGF or PTD-hEGF and microalgae transformed with the same
<130> OF17P153PCT
<150> KR 10-2016-0146839
   <151> 2016-11-04
<150> KR 10-2017-0145448
   <151> 2017-11-02
<160> 7
<170> KoPatentIn 3.0
<210> 1
   <211> 997
   <212> DNA
   <213> Phaeodactylum tricornutum
<220>
   <221> promoter
   <222> (1)..(997)
<400> 1
   tggtgccgtt gatgccgtgg ctctgcaaaa gagtcgaacg ccatgatggt atccgacggc 60
   ttgcttggtg ggagggcgga aaccttgaaa aaacgccgtc tcgtggtgtg attcggaagg 120
   caccgctttg gaattggtgg agaatagtgg aagtgtaaat gtgtttggga aggtcgagaa 180
   ccatcggaac cggtggcacg atcgccgttg gatagtagtc acattagtag cggtcctcct 240
   catgttccaa tccgttcgat cctgttcggt agcgacggaa cgaccggacg gaccggacgt 300
   gtgttgtgtt tccttttggc cttttggttt cttcgggcca gaaagagggg gtcgacggtt 360
   ggttcggcga aaggacagac agaccgattt ttgacggcgt ttggaatttg ttgtgttcaa 420
   aatttccaaa ggcaataagg aacgcaccga acaagggacc taattgctgt cgtcatcgaa 480
   gaagtaaatg cgtgacatca cagaagcggc aaagttccgc cgaggagatc ggaacttgat 540
   gaagtttgga tacgtctcgc cgtcccggaa ccagcagaat cgacgaaggc ctgccatttt 600
   gcgtagactt tcccggtgtt ggtccgtaga cggatagaga acgtacaggt atggttctac 660
   ggcgacagaa gatccaccga cgcgagagag aaattttggg gaatggatct tttggaacgg 720
   accctcagat ccgaacccga ctcaaccgac cgaagtaatc gcccccaaaa ttacagtaag 780
   tcacacacag acaacaccag tccccccaaa ggaaccccag ttgcaccaag caaaacacac 840
   acaccaaatt cgaacacgac gcaccctcgt gcaggcagca accacgatat cctgatcgat 900
   cacaacgtat aaagcgtgtg tccgcgttct gcctccctac ctaacacaca cactcgtata 960
   ctctctcata tacacaccac atcccaaact tccaagc 997
<210> 2
   <211> 243
   <212> DNA
   <213> Artificial Sequence
<220>
   <223> His-TEV-EGF
<400> 2
   atgggcagca gccaccacca ccaccaccac agcagcggcg actacgacat ccccaccggt 60
   gaaaacctgt acttccaggg caacagtgac tccgaatgcc ccctgtccca cgacggatac 120
   tgcctccacg acggtgtgtg catgtacatt gaagccttgg acaagtacgc ctgcaactgt 180
   gtcgtcggct acatcggaga acgttgtcag taccgtgacc tgaagtggtg ggaactgcgc 240
   taa 243
<210> 3
   <211> 330
   <212> DNA
   <213> Artificial Sequence
<220>
   <223> 3'-UTR
<400> 3
   cagaagcgtg ctatcgaact caaccaggga cgtgcggcac aaatgggcat ccttgctctc 60
   atggtgcacg aacagttggg agtctctatc cttccttaaa aatttaattt tcattagttg 120
   cagtcactcc gctttggttt cacagtcagg aataacacta gctcgtcttc accatggatg 180
   ccaatctcgc ctattcatgg tgtataaaag ttcaacatcc aaagctagaa cttttggaaa 240
   gagaaagaat atccgaatag ggcacggcgt gccgtattgt tggagtggac tagcagaaag 300
   tgaggaaggc acaggatgag ttttctcgag 330
<210> 4
   <211> 862
   <212> DNA
   <213> Artificial Sequence
<220>
   <223> Shble cassete
<400> 4
   acataccttc agcgtcgtct tcactgtcac agtcaactga cagtaatcgt tgatccggag 60
   agattcaaaa ttcaatctgt ttggacctgg ataagacaca agagcgacat cctgacatga 120
   acgccgtaaa cagcaaatcc tggttgaaca cgtatccttt tgggggcctc cgctacgacg 180
   ctcgctccag ctggggcttc cttactatac acagcgcgca tatttcacgg ttgccagatg 240
   tcaagatggc caagttgacc agtgccgttc cggtgctcac cgcgcgcgac gtcgccggag 300
   cggtcgagtt ctggaccgac cggctcgggt tctcccggga cttcgtggag gacgacttcg 360
   ccggtgtggt ccgggacgac gtgaccctgt tcatcagcgc ggtccaggac caggtggtgc 420
   cggacaacac cctggcctgg gtgtgggtgc gcggcctgga cgagctgtac gccgagtggt 480
   cggaggtcgt gtccacgaac ttccgggacg cctccgggcc ggccatgacc gagatcggcg 540
   agcagccgtg ggggcgggag ttcgccctgc gcgacccggc cggcaactgc gtgcacttcg 600
   tggccgagga gcaggactga accttcctta aaaatttaat tttcattagt tgcagtcact 660
   ccgctttggt ttcacagtca ggaataacac tagctcgtct tcaccatgga tgccaatctc 720
   gcctattcat ggtgtataaa agttcaacat ccaaagctag aacttttgga aagagaaaga 780
   atatccgaat agggcacggc gtgccgtatt gttggagtgg actagcagaa agtgaggaag 840
   gcacaggatg agttttctcg ag 862
<210> 5
   <211> 297
   <212> DNA
   <213> Artificial Sequence
<220>
   <223> His-TEV-PTD-EGF
<400> 5
   atgggcagca gccaccacca ccaccaccac agcagcggcg actacgacat ccccaccggt    60
   gaaaacctgt acttccaggg cagacaaatc aagatttggt tccaaaaccg tcgtatgaag   120
   tggaagaaga ccggtaacag tgactccgaa tgccccctgt cccacgacgg atactgcctc    180
   cacgacggtg tgtgcatgta cattgaagcc ttggacaagt acgcctgcaa ctgtgtcgtc   240
   ggctacatcg gagaacgttg tcagtaccgt gacctgaagt ggtgggaact gcgctaa      297
<210> 6
   <211> 4767
   <212> DNA
   <213> Artificial Sequence
<220>
   <223> EGF expression vector
<400> 6
   catatgtggt gccgttgatg ccgtggctct gcaaaagagt cgaacgccat gatggtatcc    60
   gacggcttgc ttggtgggag ggcggaaacc ttgaaaaaac gccgtctcgt ggtgtgattc   120
   ggaaggcacc gctttggaat tggtggagaa tagtggaagt gtaaatgtgt ttgggaaggt   180
   cgagaaccat cggaaccggt ggcacgatcg ccgttggata gtagtcacat tagtagcggt   240
   cctcctcatg ttccaatccg ttcgatcctg ttcggtagcg acggaacgac cggacggacc   300
   ggacgtgtgt tgtgtttcct tttggccttt tggtttcttc gggccagaaa gagggggtcg 360
   acggttggtt cggcgaaagg acagacagac cgatttttga cggcgtttgg aatttgttgt 420
   gttcaaaatt tccaaaggca ataaggaacg caccgaacaa gggacctaat tgctgtcgtc 480
   atcgaagaag taaatgcgtg acatcacaga agcggcaaag ttccgccgag gagatcggaa 540
   cttgatgaag tttggatacg tctcgccgtc ccggaaccag cagaatcgac gaaggcctgc 600
   cattttgcgt agactttccc ggtgttggtc cgtagacgga tagagaacgt acaggtatgg 660
   ttctacggcg acagaagatc caccgacgcg agagagaaat tttggggaat ggatcttttg 720
   gaacggaccc tcagatccga acccgactca accgaccgaa gtaatcgccc ccaaaattac 780
   agtaagtcac acacagacaa caccagtccc cccaaaggaa ccccagttgc accaagcaaa 840
   acacacacac caaattcgaa cacgacgcac cctcgtgcag gcagcaacca cgatatcctg 900
   atcgatcaca acgtataaag cgtgtgtccg cgttctgcct ccctacctaa cacacacact 960
   cgtatactct ctcatataca caccacatcc caaacttcca agcgaattcg atatcatggg 1020
   cagcagccac caccaccacc accacagcag cggcgactac gacatcccca ccggtgaaaa 1080
   cctgtacttc cagggcaaca gtgactccga atgccccctg tcccacgacg gatactgcct 1140
   ccacgacggt gtgtgcatgt acattgaagc cttggacaag tacgcctgca actgtgtcgt 1200
   cggctacatc ggagaacgtt gtcagtaccg tgacctgaag tggtgggaac tgcgctaatc 1260
   tagagtcgac ctgcaggcat gcaagcttca gaagcgtgct atcgaactca accagggacg 1320
   tgcggcacaa atgggcatcc ttgctctcat ggtgcacgaa cagttgggag tctctatcct 1380
   tccttaaaaa tttaattttc attagttgca gtcactccgc tttggtttca cagtcaggaa 1440
   taacactagc tcgtcttcac catggatgcc aatctcgcct attcatggtg tataaaagtt 1500
   caacatccaa agctagaact tttggaaaga gaaagaatat ccgaataggg cacggcgtgc 1560
   cgtattgttg gagtggacta gcagaaagtg aggaaggcac aggatgagtt ttctcgagac 1620
   ataccttcag cgtcgtcttc actgtcacag tcaactgaca gtaatcgttg atccggagag 1680
   attcaaaatt caatctgttt ggacctggat aagacacaag agcgacatcc tgacatgaac 1740
   gccgtaaaca gcaaatcctg gttgaacacg tatccttttg ggggcctccg ctacgacgct 1800
   cgctccagct ggggcttcct tactatacac agcgcgcata tttcacggtt gccagatgtc 1860
   aagatggcca agttgaccag tgccgttccg gtgctcaccg cgcgcgacgt cgccggagcg 1920
   gtcgagttct ggaccgaccg gctcgggttc tcccgggact tcgtggagga cgacttcgcc 1980
   ggtgtggtcc gggacgacgt gaccctgttc atcagcgcgg tccaggacca ggtggtgccg 2040
   gacaacaccc tggcctgggt gtgggtgcgc ggcctggacg agctgtacgc cgagtggtcg 2100
   gaggtcgtgt ccacgaactt ccgggacgcc tccgggccgg ccatgaccga gatcggcgag 2160
   cagccgtggg ggcgggagtt cgccctgcgc gacccggccg gcaactgcgt gcacttcgtg 2220
   gccgaggagc aggactgaac cttccttaaa aatttaattt tcattagttg cagtcactcc 2280
   gctttggttt cacagtcagg aataacacta gctcgtcttc accatggatg ccaatctcgc 2340
   ctattcatgg tgtataaaag ttcaacatcc aaagctagaa cttttggaaa gagaaagaat 2400
   atccgaatag ggcacggcgt gccgtattgt tggagtggac tagcagaaag tgaggaaggc 2460
   acaggatgag ttttctcgag gccggtctcc ctatagtgag tcgtattaat ttcgataagc 2520
   caggttaacc tgcattaatg aatcggccaa cgcgcgggga gaggcggttt gcgtattggg 2580
   cgctcttccg cttcctcgct cactgactcg ctgcgctcgg tcgttcggct gcggcgagcg 2640
   gtatcagctc actcaaaggc ggtaatacgg ttatccacag aatcagggga taacgcagga 2700
   aagaacatgt gagcaaaagg ccagcaaaag gccaggaacc gtaaaaaggc cgcgttgctg 2760
   gcgtttttcc ataggctccg cccccctgac gagcatcaca aaaatcgacg ctcaagtcag 2820
   aggtggcgaa acccgacagg actataaaga taccaggcgt ttccccctgg aagctccctc 2880
   gtgcgctctc ctgttccgac cctgccgctt accggatacc tgtccgcctt tctcccttcg 2940
   ggaagcgtgg cgctttctca atgctcacgc tgtaggtatc tcagttcggt gtaggtcgtt 3000
   cgctccaagc tgggctgtgt gcacgaaccc cccgttcagc ccgaccgctg cgccttatcc 3060
   ggtaactatc gtcttgagtc caacccggta agacacgact tatcgccact ggcagcagcc 3120
   actggtaaca ggattagcag agcgaggtat gtaggcggtg ctacagagtt cttgaagtgg 3180
   tggcctaact acggctacac tagaaggaca gtatttggta tctgcgctct gctgaagcca 3240
   gttaccttcg gaaaaagagt tggtagctct tgatccggca aacaaaccac cgctggtagc 3300
   ggtggttttt ttgtttgcaa gcagcagatt acgcgcagaa aaaaaggatc tcaagaagat 3360
   cctttgatct tttctacggg gtctgacgct cagtggaacg aaaactcacg ttaagggatt 3420
   ttggtcatga gattatcaaa aaggatcttc acctagatcc ttttaaatta aaaatgaagt 3480
   tttaaatcaa tctaaagtat atatgagtaa acttggtctg acagttacca atgcttaatc 3540
   agtgaggcac ctatctcagc gatctgtcta tttcgttcat ccatagttgc ctgactcccc 3600
   gtcgtgtaga taactacgat acgggagggc ttaccatctg gccccagtgc tgcaatgata 3660
   ccgcgagacc cacgctcacc ggctccagat ttatcagcaa taaaccagcc agccggaagg 3720
   gccgagcgca gaagtggtcc tgcaacttta tccgcctcca tccagtctat taattgttgc 3780
   cgggaagcta gagtaagtag ttcgccagtt aatagtttgc gcaacgttgt tgccattgct 3840
   acaggcatcg tggtgtcacg ctcgtcgttt ggtatggctt cattcagctc cggttcccaa 3900
   cgatcaaggc gagttacatg atcccccatg ttgtgcaaaa aagcggttag ctccttcggt 3960
   cctccgatcg ttgtcagaag taagttggcc gcagtgttat cactcatggt tatggcagca 4020
   ctgcataatt ctcttactgt catgccatcc gtaagatgct tttctgtgac tggtgagtac 4080
   tcaaccaagt cattctgaga atagtgtatg cggcgaccga gttgctcttg cccggcgtca    4140
   atacgggata ataccgcgcc acatagcaga actttaaaag tgctcatcat tggaaaacgt    4200
   tcttcggggc gaaaactctc aaggatctta ccgctgttga gatccagttc gatgtaaccc    4260
   actcgtgcac ccaactgatc ttcagcatct tttactttca ccagcgtttc tgggtgagca    4320
   aaaacaggaa ggcaaaatgc cgcaaaaaag ggaataaggg cgacacggaa atgttgaata    4380
   ctcatactct tcctttttca atattattga agcatttatc agggttattg tctcatgagc    4440
   ggatacatat ttgaatgtat ttagaaaaat aaacaaatag gggttccgcg cacatttccc    4500
   cgaaaagtgc cacctgacgt ctaagaaacc attattatca tgacattaac ctataaaaat    4560
   aggcgtatca cgaggccctt tcgtctcgcg cgtttcggtg atgacggtga aaacctctga    4620
   cacatgcagc tcccggagac ggtcacagct tgtctgtaag cggatgccgg gagcagacaa    4680
   gcccgtcagg gcgcgtcagc gggtgttggc gggtgtcggg gctggcttaa ctatgcggca    4740
   tcagagcaga ttgtactgag agtgcac                                        4767
<210> 7
   <211> 4821
   <212> DNA
   <213> Artificial Sequence
<220>
   <223> PTD-EGF expression vector
<400> 7
   catatgtggt gccgttgatg ccgtggctct gcaaaagagt cgaacgccat gatggtatcc      60
   gacggcttgc ttggtgggag ggcggaaacc ttgaaaaaac gccgtctcgt ggtgtgattc     120
   ggaaggcacc gctttggaat tggtggagaa tagtggaagt gtaaatgtgt ttgggaaggt     180
   cgagaaccat cggaaccggt ggcacgatcg ccgttggata gtagtcacat tagtagcggt     240
   cctcctcatg ttccaatccg ttcgatcctg ttcggtagcg acggaacgac cggacggacc     300
   ggacgtgtgt tgtgtttcct tttggccttt tggtttcttc gggccagaaa gagggggtcg     360
   acggttggtt cggcgaaagg acagacagac cgatttttga cggcgtttgg aatttgttgt     420
   gttcaaaatt tccaaaggca ataaggaacg caccgaacaa gggacctaat tgctgtcgtc     480
   atcgaagaag taaatgcgtg acatcacaga agcggcaaag ttccgccgag gagatcggaa     540
   cttgatgaag tttggatacg tctcgccgtc ccggaaccag cagaatcgac gaaggcctgc     600
   cattttgcgt agactttccc ggtgttggtc cgtagacgga tagagaacgt acaggtatgg     660
   ttctacggcg acagaagatc caccgacgcg agagagaaat tttggggaat ggatcttttg     720
   gaacggaccc tcagatccga acccgactca accgaccgaa gtaatcgccc ccaaaattac     780
   agtaagtcac acacagacaa caccagtccc cccaaaggaa ccccagttgc accaagcaaa     840
   acacacacac caaattcgaa cacgacgcac cctcgtgcag gcagcaacca cgatatcctg     900
   atcgatcaca acgtataaag cgtgtgtccg cgttctgcct ccctacctaa cacacacact     960
   cgtatactct ctcatataca caccacatcc caaacttcca agcgaattcg atatcatggg    1020
   cagcagccac caccaccacc accacagcag cggcgactac gacatcccca ccggtgaaaa    1080
   cctgtacttc cagggcagac aaatcaagat ttggttccaa aaccgtcgta tgaagtggaa    1140
   gaagaccggt aacagtgact ccgaatgccc cctgtcccac gacggatact gcctccacga    1200
   cggtgtgtgc atgtacattg aagccttgga caagtacgcc tgcaactgtg tcgtcggcta    1260
   catcggagaa cgttgtcagt accgtgacct gaagtggtgg gaactgcgct aatctagagt    1320
   cgacctgcag gcatgcaagc ttcagaagcg tgctatcgaa ctcaaccagg gacgtgcggc    1380
   acaaatgggc atccttgctc tcatggtgca cgaacagttg ggagtctcta tccttcctta    1440
   aaaatttaat tttcattagt tgcagtcact ccgctttggt ttcacagtca ggaataacac    1500
   tagctcgtct tcaccatgga tgccaatctc gcctattcat ggtgtataaa agttcaacat    1560
   ccaaagctag aacttttgga aagagaaaga atatccgaat agggcacggc gtgccgtatt    1620
   gttggagtgg actagcagaa agtgaggaag gcacaggatg agttttctcg agacatacct    1680
   tcagcgtcgt cttcactgtc acagtcaact gacagtaatc gttgatccgg agagattcaa    1740
   aattcaatct gtttggacct ggataagaca caagagcgac atcctgacat gaacgccgta    1800
   aacagcaaat cctggttgaa cacgtatcct tttgggggcc tccgctacga cgctcgctcc    1860
   agctggggct tccttactat acacagcgcg catatttcac ggttgccaga tgtcaagatg    1920
   gccaagttga ccagtgccgt tccggtgctc accgcgcgcg acgtcgccgg agcggtcgag    1980
   ttctggaccg accggctcgg gttctcccgg gacttcgtgg aggacgactt cgccggtgtg    2040
   gtccgggacg acgtgaccct gttcatcagc gcggtccagg accaggtggt gccggacaac    2100
   accctggcct gggtgtgggt gcgcggcctg gacgagctgt acgccgagtg gtcggaggtc    2160
   gtgtccacga acttccggga cgcctccggg ccggccatga ccgagatcgg cgagcagccg    2220
   tgggggcggg agttcgccct gcgcgacccg gccggcaact gcgtgcactt cgtggccgag    2280
   gagcaggact gaaccttcct taaaaattta attttcatta gttgcagtca ctccgctttg    2340
   gtttcacagt caggaataac actagctcgt cttcaccatg gatgccaatc tcgcctattc    2400
   atggtgtata aaagttcaac atccaaagct agaacttttg gaaagagaaa gaatatccga    2460
   atagggcacg gcgtgccgta ttgttggagt ggactagcag aaagtgagga aggcacagga    2520
   tgagttttct cgaggccggt ctccctatag tgagtcgtat taatttcgat aagccaggtt    2580
   aacctgcatt aatgaatcgg ccaacgcgcg gggagaggcg gtttgcgtat tgggcgctct    2640
   tccgcttcct cgctcactga ctcgctgcgc tcggtcgttc ggctgcggcg agcggtatca    2700
   gctcactcaa aggcggtaat acggttatcc acagaatcag gggataacgc aggaaagaac    2760
   atgtgagcaa aaggccagca aaaggccagg aaccgtaaaa aggccgcgtt gctggcgttt 2820
   ttccataggc tccgcccccc tgacgagcat cacaaaaatc gacgctcaag tcagaggtgg 2880
   cgaaacccga caggactata aagataccag gcgtttcccc ctggaagctc cctcgtgcgc 2940
   tctcctgttc cgaccctgcc gcttaccgga tacctgtccg cctttctccc ttcgggaagc 3000
   gtggcgcttt ctcaatgctc acgctgtagg tatctcagtt cggtgtaggt cgttcgctcc 3060
   aagctgggct gtgtgcacga accccccgtt cagcccgacc gctgcgcctt atccggtaac 3120
   tatcgtcttg agtccaaccc ggtaagacac gacttatcgc cactggcagc agccactggt 3180
   aacaggatta gcagagcgag gtatgtaggc ggtgctacag agttcttgaa gtggtggcct 3240
   aactacggct acactagaag gacagtattt ggtatctgcg ctctgctgaa gccagttacc 3300
   ttcggaaaaa gagttggtag ctcttgatcc ggcaaacaaa ccaccgctgg tagcggtggt 3360
   ttttttgttt gcaagcagca gattacgcgc agaaaaaaag gatctcaaga agatcctttg 3420
   atcttttcta cggggtctga cgctcagtgg aacgaaaact cacgttaagg gattttggtc 3480
   atgagattat caaaaaggat cttcacctag atccttttaa attaaaaatg aagttttaaa 3540
   tcaatctaaa gtatatatga gtaaacttgg tctgacagtt accaatgctt aatcagtgag 3600
   gcacctatct cagcgatctg tctatttcgt tcatccatag ttgcctgact ccccgtcgtg 3660
   tagataacta cgatacggga gggcttacca tctggcccca gtgctgcaat gataccgcga 3720
   gacccacgct caccggctcc agatttatca gcaataaacc agccagccgg aagggccgag 3780
   cgcagaagtg gtcctgcaac tttatccgcc tccatccagt ctattaattg ttgccgggaa 3840
   gctagagtaa gtagttcgcc agttaatagt ttgcgcaacg ttgttgccat tgctacaggc 3900
   atcgtggtgt cacgctcgtc gtttggtatg gcttcattca gctccggttc ccaacgatca 3960
   aggcgagtta catgatcccc catgttgtgc aaaaaagcgg ttagctcctt cggtcctccg 4020
   atcgttgtca gaagtaagtt ggccgcagtg ttatcactca tggttatggc agcactgcat 4080
   aattctctta ctgtcatgcc atccgtaaga tgcttttctg tgactggtga gtactcaacc 4140
   aagtcattct gagaatagtg tatgcggcga ccgagttgct cttgcccggc gtcaatacgg 4200
   gataataccg cgccacatag cagaacttta aaagtgctca tcattggaaa acgttcttcg 4260
   gggcgaaaac tctcaaggat cttaccgctg ttgagatcca gttcgatgta acccactcgt 4320
   gcacccaact gatcttcagc atcttttact ttcaccagcg tttctgggtg agcaaaaaca 4380
   ggaaggcaaa atgccgcaaa aaagggaata agggcgacac ggaaatgttg aatactcata 4440
   ctcttccttt ttcaatatta ttgaagcatt tatcagggtt attgtctcat gagcggatac 4500
   atatttgaat gtatttagaa aaataaacaa ataggggttc cgcgcacatt tccccgaaaa 4560
   gtgccacctg acgtctaaga aaccattatt atcatgacat taacctataa aaataggcgt 4620
   atcacgaggc cctttcgtct cgcgcgtttc ggtgatgacg gtgaaaacct ctgacacatg       4680
   cagctcccgg agacggtcac agcttgtctg taagcggatg ccgggagcag acaagcccgt       4740
   cagggcgcgt cagcgggtgt tggcgggtgt cggggctggc ttaactatgc ggcatcagag       4800
   cagattgtac tgagagtgca c                                                 4821